# EUROPEAN PATENT SPECIFICATION

(11) **EP 2 329 785 B1**
(45) Date of publication and mention of the grant of the patent: **06.03.2013**
(21) Application number: 10075566.9
(22) Date of filing: 24.01.2005
(51) Int. Cl.: A61B 18/22, A61B 17/22, A61B 17/32, B23K 26/14, A61B 17/3203, A61C 1/00

(54) **Electromagnetically induced treatment devices**
Elektromagnetisch induzierte Behandlungsvorrichtungen
Dispositifs de traitement induit électromagnétiquement

(30) Priority: 22.01.2004 US 538200
(43) Date of publication of application: 08.06.2011
(62) Divisional of application: 05722585.6
(73) Proprietor: Biolase, Inc., Irvine, CA 92618 (US)
(72) Inventor: Boutoussov, Dmitri, California 92629 (US)
(74) Representative: Weber-Bruls, Dorothée

(56) References cited:
- WO-A-01/39682
- US-B1- 6 231 567
- US-B1- 6 254 597
- US-B1- 6 561 803

## Description

### BACKGROUND OF THE INVENTION

### 1. Field of the Invention

The present invention relates generally to treatment devices and, more particularly, to devices which use electromagnetic energy and which can cut, ablate or otherwise treat a medical, dental, industrial, or other target surface.

### 2. Description of Related Art

Treatment devices have existed in the prior art that harness electromagnetic energy in some way to treat a target surface. For example, mechanical drills and optical cutters, both of which utilize electromagnetic energy in some way, are well known in medical, dental, and industrial settings for treating target surfaces. For instance, dental optical cutters can employ a source of electromagnetic energy, such as a laser source, with an optical fiber system that is connected to the laser. source and configured to direct radiation from the laser through one or more optical fibers to a tooth surface to be cut.

The U.S. Patent 6,231,567 B1 discloses a device for treating a tissu by the interaction of electromagnetic energy with fluid particles comprising a treatment tip a a plurality of fluid outputs.

### SUMMARY OF THE INVENTION

An electromagnetically induced treatment device includes a body member having a distal end, an optic guide extending from the body member distal end, and at least three nozzles positioned around the optic guide to provide a volume of atomized fluid particles in proximity to the distal end of the optic guide. The treatment device may also include a mixing chamber proximally located to the nozzles.

Another electromagnetically induced treatment device includes a body member having a distal end and at least three nozzles located at the distal end. This embodiment does not necessarily have an optic guide extending from the body member distal end. The at least three nozzles are effective to provide a volume of atomized fluid particles spaced away from the body member distal end. The electromagnetically induced treatment device includes an energy output to direct energy toward the volume of atomized fluid particles. This treatment device may also include a mixing chamber proximally located to the nozzles.

Any feature or combination of features described herein are included within the scope of the present invention provided that the features included in any such combination are not mutually inconsistent as will be apparent from the context, this specification, and the knowledge of one of ordinary skill in the art. For purposes of summarizing the present invention, certain aspects, advantages and novel features of the present invention have been described herein. Of course, it is to be understood that not necessarily all such aspects, advantages or features will be embodied in any particular embodiment of the present invention. Additional advantages and aspects of the present invention are apparent in the following detailed description.

The invention is as defined by the appended set of claims.

### BRIEF DESCRIPTION OF THE DRAWINGS

FIG.1A is an illustration of an optical cutter having less than three nozzles at a first intensity.
FIG. 1B is an illustration of the optical cutter of FIG. 1A at a second intensity.
FIG. 2A is an illustration of an optical treatment device having three nozzles emitting atomized fluid particles at a first intensity.
FIG. 2B is an illustration of the optical treatment device of FIG. 2A emitting atomized fluid particles at a second intensity.
FIG. 2C is an illustration of the optical treatment device of FIG. 2A emitting atomized fluid particles at a third intensity.
FIG. 3 is a cross-sectional view of an optical treatment device having three nozzles.
FIG. 4 is a perspective view of the optical treatment device of FIG. 3.
FIG. 5 is a cross-sectional view, taken along the line 5-5' of the optical treatment device of FIG. 4. The cross-sectional view corresponds to that of FIG. 3 but without the cutting or treatment tip and the ferrule.

### DETAILED DESCRIPTION OF THE ILLUSTRATED EMBODIMENTS

Reference will now be made in detail to certain embodiments of the invention, examples of which are illustrated in the accompanying drawings. Wherever possible, the same or similar reference numbers are used in the drawings and the description to refer to the same or like parts. It should be noted that the drawings are in simplified form and are not to precise scale. In reference to the disclosure herein, for purposes of convenience and clarity only, directional terms, such as, top, bottom, left, right, up, down, over, above, below, beneath, rear, and front, are used with respect to the accompanying drawings. Such directional terms should not be construed to limit the scope of the invention in any manner.

Although the disclosure herein refers to certain illustrated embodiments, it is to be understood that these embodiments are presented by way of example and not by way of limitation. The intent of the following detailed description, although discussing exemplary embodiments, is to be construed to cover all modifications, alternatives, and equivalents of the embodiments as may fall within the spirit and scope of the invention as defined by the appended claims.

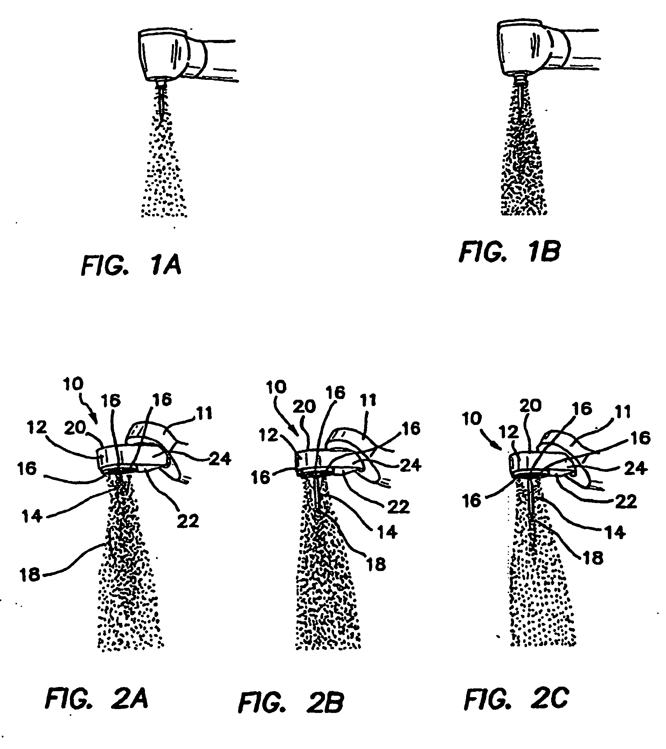

Referring more particularly to the drawings, FIGS. 1A and 1B illustrate electromagnetically induced cutters that have less than three nozzles positioned in proximity to an optic guide.

In accordance with the disclosure herein, FIGS. 2A-2C show an exemplary embodiment of an electromagnetically induced treatment device 10 of the present invention that is adapted to facilitate performance of a procedure. Treatment device 10 uses electromagnetic energy and can be adapted to cut, ablate or otherwise treat a medical, dental, industrial, or other target surface. The treatment device 10 is shown as comprising a body 11, such as a hand-held body, having a distal end 12. The distal end 12 of the body 11 includes a cutting or treatment fiber, such as a fiber optic guide 14, and at least three nozzles 16. The fiber optic guide 14 is typically coupled to a laser energy source so that energy may be emitted from the distal end of the fiber optic guide 14. The treatment device 10 typically includes an air tube (i.e., a tube for delivering a gaseous composition) and/or a liquid tube (i.e., a tube for delivering fluid and/or liquid) that, in an illustrated embodiment provide air and liquid to the nozzles 16. The treatment device 10 may include one or more air tubes or liquid tubes in any combination. The nozzles 16 are positioned in the treatment device to direct a mixture of the gas (e.g., air) and fluid (e.g., liquid) away from the distal end 12. In the illustrated embodiment, the nozzles 16 are positioned to direct the air and liquid mixture towards a distal end of the fiber optic guide 14.

The liquid tube may be configured to pass any suitable fluid or liquid, such as water or, for example, other water-based liquids, toward the nozzles 16. In certain embodiments the fluids may be conditioned such as disclosed in U.S. Patent Nos. 5,785,521, 6,350,123 and 6,561,803, and U.S. Provisional Application filed January 19,2005 and entitled FLUID CONDITIONING SYSTEM.

The air and liquid mixture may then interact with, for example, laser energy emitted from the fiber optic guide 14 to create an interaction zone 18. Examples of treatment devices and additional components which may be used in accordance with the disclosure herein include those identified in U.S. Patent No. 5,741,247, U.S. Patent No. 6,254,597, U.S. Application filed January 10, 2005 and entitled ELECTROMAGNETIC ENERGY DISTRIBUTIONS FOR ELECTROMAGNETICALLY INDUCED DISRUPTIVE CUTTING.

As shown in FIG. 2A, the treatment device 10 includes at least three fluid outputs, which in the illustrated embodiment comprise three nozzles 16. In additional embodiments, the treatment device 10 may include more than three nozzles. The nozzles 16 are illustrated as being located around the optic guide 14. When more than three nozzles are provided, the nozzles may be arranged in a substantially circular configuration to surround the optic guide 14. In certain embodiments, the nozzles may be provided as a nozzle ring that substantially surrounds the optic guide. In an embodiment having a nozzle ring, a single nozzle, or a plurality of arc shaped nozzles, circumvents the optic guide. 14. In the embodiment of FIG. 2, the nozzles 16 may be evenly spaced around the optic guide 14, for example, at zero, one hundred twenty, and two hundred forty degrees, or may be spaced at irregular intervals. In certain embodiments, each nozzle may be spaced from another nozzle by a distance of about 5 mm. The nozzles may be located at (e.g., flush with) a surface of the distal end of the optical treatment device, or they may extend away from the surface of the optical treatment device distal end. In certain embodiments of treatment devices having a nozzle ring, the nozzle or nozzles may be located within the body and the ring may be positioned at the surface of the optical treatment device distal end. Providing three or more nozzles on the treatment device 10 may be effective to create a finer spray of particles (e.g., enhanced or better atomization of the particles) relative to cutters that have less than three nozzles.

The nozzles 16 or nozzle ring are positioned on the treatment device 10 to provide, in the illustrated embodiment, a mist or spray of, for example, atomized fluid particles around the optic guide 14. The nozzles are typically relatively small in size. In exemplary embodiments, the nozzles have an outlet diameter between about 100 micrometers and about 500 micrometers. In an exemplary embodiment, the nozzles 16 are provided at angles so that primary axes of expelled air and liquid of the nozzles intersect at a distance of about 5 mm from the nozzles or, in an alternative embodiment, at the same or a lesser distance from the distal end of the optic guide 14. In an exemplary embodiment, energy emitted from the optic guide 14 interacts with the atomized fluid particles to cause at least a portion of the particles to expand. The expansion of the particles can be effective to impart cutting and/or ablating forces onto a target surface.

In certain embodiments, air may be directed to the nozzles 16 at a pressure ranging from about 5 pounds per square inch (psi) to about 60 psi. The air may be directed to the nozzles 16 at a flow rate ranging from about 0.5 liters/minute to about 20 liters/minute. However, in at least one embodiment, the air may have a flow rate of about 0.001 liters/minute.

In certain embodiments, the liquid may be directed to the nozzles at a pressure ranging from about 5 psi to about 60 psi and a flow rate of about 2 ml/minute to about 100 ml/minute. In at least one embodiment, the liquid flows at a rate of about 0.001 ml/minute. In an exemplary embodiment, the liquid comprises water.

As one example, a treatment device in accordance with the disclosure herein includes an air flow tube containing air flowing at about 2 1/micute at a pressure of about 20 psi and a liquid (e.g., water) flow tube containing water flowing at about 40 ml/minute at a pressure of about 20 psi.

The treatment device may also be provided with a mixing chamber, which may be positioned upstream or proximal to the nozzles 16. The chamber may be effective to promote the mixing of the air and liquid before it is emitted from the nozzles 16. By providing a mixing chamber, it is possible to obtain a desired cutting of a target surface with little air or gas. For example, as shown in FIGS. 2A-2C, a desired amount of cutting may be obtained with a mixture of about 5-15% of air to about 50% of water. More particularly, in the illustrated examples, the cutters of FIGS. 2A-2C utilize mixtures of 15% air to 50% water, 10% air to 50% water, and 5% air to 50% water, respectively. In comparison, the cutters shown in FIGS. 1A and 1B utilize a mixture of 65% air to 55% water, or 90% air to 75% water.

In one embodiment, a water flow rate through the liquid line of the treatment device 10 may be about 84 ml/minute (e.g., 100%), and an air flow rate through the gas line of the treatment device may be 13 liters/minute (e.g., 100%). Thus, the values shown in FIGS. 2A-2C may be understood in reference to such flow rates. The cutting effects may be substantially linear from 0% to about 100% for each of the gas and liquid lines. As shown in the accompanying drawings, the treatment device 10 shown in FIGS. 2A-2C can obtain a desired cutting effect using less air relative to the cutter shown in FIGS. 1A and 1B.

The distal end 12 of the treatment device may be provided as a retractable portion that is configured to be retracted into and displaced from the treatment device body 11. The distal end 12 may have a first or top surface 20 and a second opposing or bottom surface 22. The nozzles 16 and optic guide 14 may be provided, for example, on the second surface 22. The first and second surface may be connected by a sidewall 24 that is, for example, substantially straight at a proximal region and curved at a distal end In one embodiment, the nozzles may be provided on a rotating disk element which is effective to rotate the nozzles around the optic guide to generate different cutting effects relative to an orientation of the treatment device 10.

Intense energy is emitted from the fiber optic guide. This intense energy can be generated from a coherent source, such as a laser, or any other type of electromagnetic energy radiating source and/or excitation source. In the illustrated embodiment comprising a laser, a flashlamp can be used to stimulate a laser rod to thereby generate coherent optical radiation. Other means, however, are also contemplated by the present invention. Diodes, for example, may be used instead of flashlamps with the excitation source. The use of diodes for generating light amplification by stimulated emission is discussed in the book Solid-State Laser Engineering, Fourth Extensively Revised and Updated Edition, by Walter Koechner, published in 1996, the contents of which are expressly incorporated herein by reference.

In an illustrated embodiment, the laser comprises either an erbium, chromium, yttrium, scandium, gallium garnet (Er, Cr.YSGG) solid state laser, which generates electromagnetic energy having a wavelength in a range of 2.70 to 2.80 microns, or an erbium, yttrium, aluminum garnet (Er:YAG) solid state laser, which generates electromagnetic energy having a wavelength of 2.94 microns. As presently embodied, the Er, Cr.YSGG solid state laser can have a wavelength of approximately 2.78 microns and the Er:YAG solid state laser can have a wavelength of approximately 2.94 microns.

Although the fluid emitted from the nozzles 16 may be aqueous based, other fluids may be used and appropriate wavelengths of the electromagnetic energy source may be selected to allow for, in some embodiments, high absorption by the fluid. Another possible laser system can include a chromium, thulium, erbium, yttrium, aluminum garnet (CTE:YAG) solid state laser, which generates electromagnetic energy having a wavelength of 2.69 microns. The actual fluid used may vary as long as it is properly matched (meaning it is highly absorbed, in an exemplary embodiment) to the selected electromagnetic energy source (e.g., laser) wavelength.

When atomized fluid particles are used, cutting forces can be imparted when the particles absorb electromagnetic energy within the interaction zone. A delivery system for delivering the electromagnetic energy can include a fiber optic energy guide or equivalent which attaches to the laser system and travels to the desired work site. Fiber optics or waveguides are typically long, thin and lightweight, and are easily manipulated. Fiber optics can be made of calcium fluoride (CaF), calcium oxide (CaO₂), zirconium oxide (ZrO₂), zirconium fluoride (ZrF), sapphire, hollow waveguide, liquid core, TeX glass, quartz silica, germanium sulfide, arsenic sulfide, germanium oxide (GeO₂), and other materials. Other delivery systems in addition to or as an alternative to optic guide 14 can include devices comprising mirrors, lenses and other optical components where the energy travels through a cavity, is directed by various mirrors, and is focused onto the targeted cutting site with specific lenses. The illustrated embodiment of light delivery for medical applications of the present invention is through a fiber optic conductor (e.g., optic guide 14).

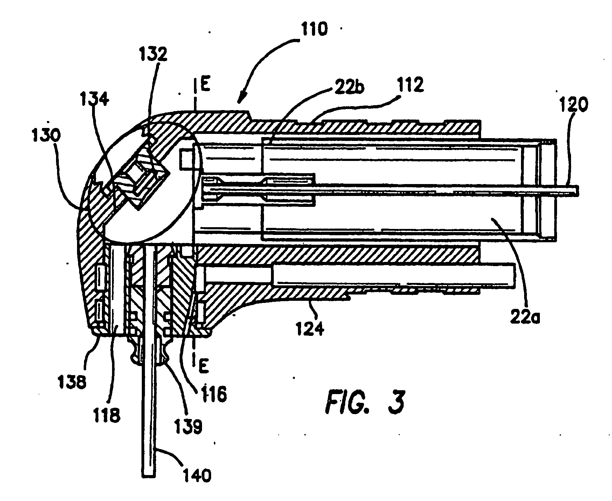

FIG. 3 is a cross-sectional view of an optical treatment device having three nozzles. Optical treatment device 110 includes an elongate body 112 having a generally tube-like structure with a hollow interior that is structured to contain a plurality of light transmitters, such as optical fibers and the like, which are used to transmit light toward or from a handpiece. Optical treatment device 110 comprises a distal end having a distal portion 124 and a proximal end (not shown), the distal end being defined as the output end furthest from an operator and closest to a target surface. Elongate body 112 can be a hollow structure having a proximal portion (not shown) that is flexible. Elongate body 112 can be made from any suitable material or materials, such as stainless steel, metal coil or plastic. In addition, optical treatment device 110 is illustrated as having a generally cylindrical cross-section, but it could also include one or more portions with different cross-sectional shapes including oval, rectangular, or triangular, and the like.

Optical treatment device 110 can comprise a plurality of proximal members, each having a hollow interior configured to accommodate one or more light transmitters or other tubular or elongate structures that have cross-sectional areas less than the cross-sectional area of the hollow interior. The proximal members can be arranged such that the hollow interiors of each proximal member is in communication with the hollow interior of elongate body 112. This arrangement can provide a substantially continuous path for the light transmitters to extend from the proximal end to the distal end of elongate body 112. An exemplary embodiment can comprise four proximal members, but additional embodiments can comprise two, or three or more proximal members, depending on for example the number of light transmitters being used in the optical treatment device. Two of the proximal members 22A and 22B can be provided with substantially equal diameters, with another one of the proximal members having a diameter that is different than either of the diameters of the other two proximal members. Other diameter distributions among the four proximal members of the exemplary embodiment may be implemented in modified embodiments. According to the exemplary embodiment of FIG. 3, the fourth one of the proximal members can be formed as or provided with, for example, a cutting or treatment fiber 120 for transmitting, in the case of a cutting application, cutting electromagnetic (e.g., laser) energy.

Optical treatment device 110 is illustrated as being configured to be held by a hand of a user. In a preferred embodiment, optical treatment device 110 is configured to direct electromagnetic energy from a handpiece and/or receive energy that may be generated in proximity to the handpiece. The optical treatment device can be used in medical, industrial, dental, and other applications. In one embodiment, the optical treatment device is a device for emitting electromagnetic energy in dental applications. The electromagnetic energy preferably includes light, such as visible light, laser light, and the like. The device can be used in dental hygiene procedures as well. Optical treatment device 110 is typically connected to at least one external electromagnetic energy source, such as a laser, a light emitting diode (LED), and/or a lamp, so that the electromagnetic energy that is generated by the source can be transmitted through optical treatment device 110 and directed from a handpiece.

In the illustrated embodiment of elongate body 112, the distal end includes an electromagnetic energy emitting output end, and the proximal end includes an electromagnetic energy input end. Each of the proximal members can include a humen dimensioned to accommodate one or more light transmitters or other tube- or fiber-like structures, with, for example, each of the first three of the mentioned proximal members containing three energy emitting fibers, such as optical fibers, and the fourth proximal member containing, for example, one cutting or treatment fiber 120 such as a power erbium fiber. The energy emitting fibers can be manufactured, for example, from plastic using conventional techniques, such as extrusion and the like.

At the proximal end, fibers of the first two or three proximal members are configured to receive and transmit light from for example a laser, an LED, or a lamp. As presently embodied, white light, for example white light generated by one or more white light LEDs is input. In an exemplary embodiment, two ultra-bright white light LEDs can be used as a source of illumination light for transmission through the fibers, with each LED generating, for example, electromagnetic energy at a power level of about 200 mW in either continuous wave (CW) or pulsed mode. In other embodiments, one or both white light LEDs can be substituted with different LEDs having different properties such as different colors (e.g., blue). Blue light can be particularly useful in curing dental composites, tooth whitening, and caries detection, among other things, when the device is used for dental care and hygiene. In this case, each of the proximal member coupled to the blue light may include an optional shutter mechanism or filter to influence the transmission of the blue light. The shutter mechanism or filter may be structured to comprise, for example, phosphoric filters, for converting blue light into white, or may comprise other components or configurations for converting the input light to any other visible light.

A third one of the first three proximal members can be configured to accommodate three optical fibers that are configured to collect or receive reflected and scattered light from the output end of optical treatment device 110 and guide that light back toward the proximal end. The reflected and/or scattered light can be used as a feedback signal, which can be passed to a sensor or other suitable device for analysis. The feedback signal can facilitate, for example, detection of damage to an optical surface (e.g., aiming red light beam will scatter and reflect back) or fluorescence of dental material (e.g., caries, bacteria, demineralization, and the like).

At the output end of elongate body 112, light is emitted from and collected into optical treatment device 110. In the illustrated embodiment, light or other electromagnetic radiation is emitted from, for example, at least a plurality of the energy emitting fibers, and light is collected by a transparent tip or other type(s) of waveguide(s) for routing back to, for example, the proximal end.

Electromagnetic radiation within the cutting or treatment fiber 120 can be derived from an erbium, chromium, yttrium scandium gallium garnet (Er, Cr.YSGG) solid state laser, which generates electromagnetic energy having a wavelength of approximately 2.78 microns at an average power of up to 8 Watts, a repetition rate of about 10 to 50 Hz, and a pulse width of about 150 to 700 microseconds. Moreover, the electromagnetic radiation may further comprise an aiming beam, such as light having a wavelength of about 655 nm and an average power of about 1 mW (CW or pulsed mode). Emitted light can be directed toward a working or target surface, such as a tissue surface, including a surface of a tooth, to perform one or more light sensitive procedures.

In one embodiment, optical treatment device 110 comprises a light guide path with a bend, which as presently embodied comprises an approximately ninety degree bend, wherein a light path altering structure, such as a reflector 130, is provided. A portion of optical treatment device 110 disposed distally of phantom line E-E' in FIG. 3 can thus in some embodiments be rotated about a longitudinal axis of the proximal portion of optical treatment device 110 that is disposed proximally of the phantom line E-E'. Details and functions pertaining to such a structure, which can facilitate a rotating-handpiece operation, are disclosed in U.S. Patent No. 6,389,193 and U.S. Provisional Application No. 60/589,536, filed July 20, 2004 and entitled CONTRA-ANGLE ROTATING HANDPIECE HAVING TACTILE-FEEDBACK. TIP FERRULE, the entire contents of both which are expressly incorporated herein by reference. With continuing reference to FIG. 3, reflector 130 is illustrated as including a plurality of mirrors 132 and 134. In other embodiments, fewer or more mirrors and/or additional or alternative structures may be provided. Mirror 132 is illustrated as being configured to alter the light emitted from the cutting or treatment fiber 120, and mirror 134 is illustrated as being configured to alter the path of light emitted from one or more of the energy emitting fibers. In addition, mirror 134 can be configured to direct light that is reflected back from the target surface toward the proximal end of the optical treatment device 110 to provide a signal that can be used for, as an example, analysis, as discussed above.

Optical treatment device 110 is also illustrated as including a cutting or treatment tip 140 to direct light toward a target surface. In addition, a sleeve 138 is provided that substantially surrounds cutting or treatment tip 140. The sleeve 138 can be made of a material that is substantially transparent to permit light emitted from energy emitting fibers, such as white light, to be directed into and transmitted through the sleeve 138 toward a target surface.

The sleeve 138 is provided with three or more nozzles 116 (cf. 16), each of which is, independently of the others, be disposed, in a radial dimension, at least partially within the sleeve 138, and which further may be disposed, in an output dimension that is parallel to an axis of the cutting or treatment fiber 140, near a proximal, distal, or any other portion or portions of the sleeve 138, or at any other location or locations along the output dimension. The sleeve 138 can be mounted into or around, the ferrale139 and is provided with multiple openings 118 and/or 119 for optical waveguides to transmit light In other embodiments the sleeve 138 may be constructed of transparent material such as sapphire or clear plastic with a few or all of the optical waveguide openings omitted.

Light from energy transmitting fibers may be used, for example, to illuminate the target surface. The illumination of the target surface may occur continuously during a procedure being performed, or the illumination may be interrupted. In addition, the illumination may be automatically or manually controlled. Mirrors 132 and 134 may be constructed to focus one or more light beams into cutting or treatment tip 140. In the illustrated embodiment, mirror 132 is constructed to focus the erbium laser beam emitted from the cutting or treatment fiber 120 into cutting or treatment tip 140, and mirror 134 is constructed to focus the light emitted from energy emitting fibers, such as blue light, white light, or other light, into the ferrule 139 and/or sleeve. 138.

Optical treatment device 110 may also include a tip structure, such as a curing tip. The other tip structure can be used instead of, or in conjunction with, cutting or treatment tip 140 and/or sleeve138. When the tip structure is a curing tip, the curing tip can be positioned in optical treatment device 110 and configured to receive or collect blue light emitted from the energy emitting fibers to direct the blue light toward a target surface to obtain a desired effect, such as curing of dental composites. To increase the amount of blue light that is collected by tip structure, a diameter can be chosen for the tip structure to maximize the amount of blue light collected. Cutting or treatment tip 140 and tip structure can be made of a sapphire or glass materials, including plastic materials, that is/are optically transparent to permit the light to be effectively transmitted therethrough to a target surface.

The cutting or treatment tip, ferrule, and/or associated structure may be configured, modified and/or adapted, in whole or in part, as described in U.S. Provisional Application filed July 20, 2004 and entitled CONTRA-ANGLE ROTATING HANDPIECE HAVING TACTILE-FEEDBACK TIP FERRULE, U.S. Application , filed January 10, 2005 and entitled MODIFIED-OUTPUT FIBER OPTIC TIPS, and U.S. Application No. , filed January 10, 2005 and entitled ILLUMINATION DEVICE AND RELATED METHODS.

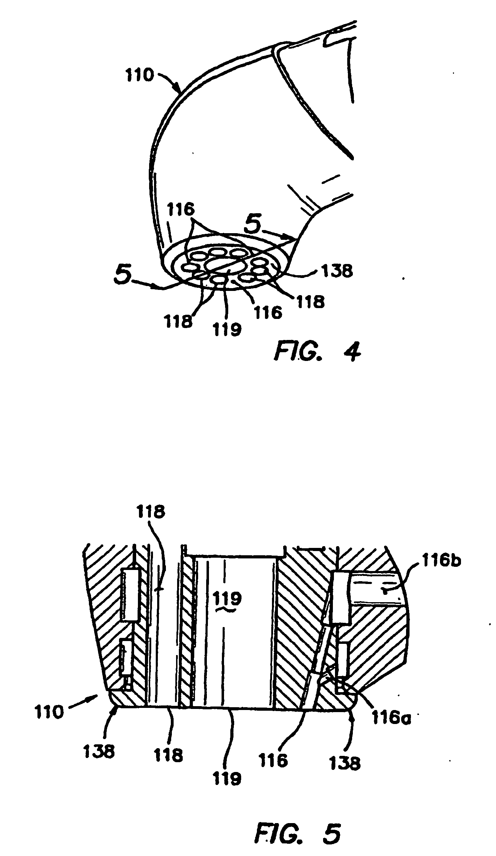

FIG. 4 is a perspective view of the optical treatment device of FIG. 3. FIG. 5 is a cross-sectional view, taken along the line 5-5' of the optical treatment device of FIG. 4. The cross-sectional view corresponds to that of FIG. 3 but without the cutting or treatment tip and the ferrule. In the illustrated embodiment of FIG. 5, a gas (e.g., air) line 116a and a liquid (e.g., water) line 116b are coupled to supply each nozzle 116.

According to an exemplary embodiment, materials are removed from a target surface by cutting forces other than purely conventional thermal cutting forces. Laser energy can be used in combination with output from nozzles 16 to induce cutting and/or ablating forces onto and/or within the targeted material. In accordance with one cutting mechanism, which is not mutually exclusive of others, the atomized fluid particles act as a medium for transforming at least part of the electromagnetic energy of the laser into disruptive cutting and/or ablating forces.

The treatment device 10 disclosed herein may be used to cut or remove biological or non-biological materials. Biological materials may include hard and soft tissues. Biological materials can include plaque, tartar, a biological layer or film of organic consistency, a smear layer, a polysaccharide layer, and a plaque layer. A smear layer may comprise fragmented biological material, including proteins, and may include living or decayed items, or combinations thereof. A polysaccharide layer may comprise a colloidal suspension of food residue and saliva. Plaque refers to a film including food and saliva, which often traps and harbors bacteria therein. These layers or films may be disposed on teeth, other biological surfaces, and nonbiological surfaces. For example, the treatment device 10 may be used to remove dental material from a patient's teeth, such as by removing tooth enamel, tooth dentin, tooth cementum, tooth decay, amalgam, composites materials, tarter and calculus. The treatment device 10 may also be used to cut or remove bone, cartilage, or portions thereof. Or, the treatment device 10 may be used to cut soft tissues such as fat, skin, mucosa, gingiva, muscle, heart, liver, kidney, brain, eye, and vessels. The term "fat" refers to animal tissue consisting of cells distended with greasy or oily matter. Other soft tissues such as breast tissue, lymphangiomas, and hemangiomas are also contemplated.

Nonbiological materials may include glass and semiconductor chip surfaces, for example. The electromagnetically induced cutting mechanism can be further be used to cut or ablate ceramics, cements, polymers, porcelain, and implantable materials and devices including metals, ceramics, and polymers. The electromagnetically induced cutting mechanism can also be used to cut or ablate surfaces of metals, plastics, polymers, rubber, glass and crystalline materials, concrete, wood, cloth, paper, leather, plants, and other man-made and naturally occurring materials. Metals can include, for example, aluminum, copper, and iron.

Thus, in accordance with the disclosure herein and in one embodiment, an apparatus for imparting cutting and/or ablating forces onto, near, and/or within a target surface includes at least three nozzles for placing atomized fluid particles into an interaction zone near the target surface, and an optic guide for directing electromagnetic energy from an energy source into the interaction zone. Additionally one or more controls can be configured to permit a user to control the cutting effects provided by the apparatus. As discussed herein, at least a portion of the particles can absorb the energy emitted from the optic guide to create cutting and/or ablating forces near, on, and/or within the target surface and/or provide cooling. As discussed herein, another embodiment includes a nozzle ring instead of or in addition to the at least three nozzles.

The above-described embodiments have been provided by way of example, and the present invention is not limited to these examples. Multiple variations and modification to the disclosed embodiments will occur, to the extent not mutually exclusive, to those skilled in the art upon consideration of the foregoing description. Additionally, other combinations, omissions, substitutions and modifications will be apparent to the skilled artisan in view of the disclosure herein.

## Claims

1. A treatment device (110) comprising an excitation source of electromagnetic energy for facilitating performance of a procedure on a target, comprising:
(a) a cutting or treatment tip;
(b) one or more light transmitters;
(c) a sleeve (138) having optical waveguides (118) which surrounds the cutting or treatment tip and which is capable of transmitting light emitted from the one or more light transmitters in a direction corresponding to that of electromagnetic energy directed by the cutting or treatment tip from the output distally away from the treatment device (110) and into a volume distally disposed of the treatment device (110) to illuminate the target surface;
(d) at least three fluid outputs (116) arranged within the sleeve (138), pointed away from the treatment device (110) toward the volume, and configured to propagate fluid distally away from the treatment device (110) toward the volume to thereby place the propagating fluid into the electromagnetic energy, within the volume, as the electromagnetic energy is directed distally away from the treatment device into the volume.

2. The treatment device (110) as set forth in claim 1, wherein:
the fluid outputs (116) are disposed around the cutting or treatment tip on a planar surface normal to the direction of the electromagnetic energy; and
the cutting or treatment tip outputs the electromagnetic energy relative to the placement of the propagating fluid so that the electromagnetic energy intersects with the propagating fluid, in a form of at least one output pulse which during use intersects with the propagating fluid in the volume.

3. The treatment device (110) as set forth in claims 1 or 2, wherein:
the fluid outputs (116) are disposed about the cutting or treatment tip on a surface normal to the direction;
each of the fluid outputs (116) is oriented with a longitudinal axis parallel to the direction for outputting fluid particles along the axis; and
the electromagnetic energy imparts a relatively large amount of energy into at least part of the propagating fluid in the volume, the relatively large amount of energy imparted into the propagating fluid being sufficient to cause the propagating fluid to expand and apply disruptive cutting or ablating forces onto the target.

4. The treatment device as set forth in any of claims 1-3, wherein the plurality of illumination waveguides (118) in the sleeve (138) are each disposed adjacent to one or more of the fluid outputs (116).

5. The treatment device (110) as set forth in any of claims 1-4, wherein:
the fluid outputs (116) are arranged on a planar surface (138); and
the surface (138) comprises the plurality of waveguides (118), each being interspaced between a corresponding two of the fluid outputs (116).

6. The treatment device (110) as set forth in any of claims 1-5, wherein:
the fluid outputs (116) are configured to place water into the volume; and
the cutting or treatment tip receives electromagnetic energy from one of an Er: YAG, an Er.YSGG, an Er, Cr:YSGG and a CTE:YAG laser.

7. The treatment device (110) as set forth in any of claims 1-6, wherein the plurality of waveguides (118) disposed in the sleeve (138) is configured for outputting the visible light, and the fluid outputs (116) are configured to place, simultaneously with the directing of electromagnetic energy from the cutting or treatment tip and the outputting of light from the waveguides (118), fluid into the volume.

8. The treatment device (110) as set forth in any of claims 1-7, wherein the electromagnetic energy from the cutting or treatment tip has one of (a) a wavelength within a range from about 2.69 to about 2.80 microns and (b) a wavelength of about 2.94 microns.

9. The treatment device (110) as set forth in any of claims 1-8, wherein the fluid outputs (116) are circumferentially arranged and the plurality of waveguides (118) is circumferentially disposed on the surface for outputting light.

10. The treatment device (110) as set forth in any of claims 1-9, wherein the plurality of waveguides (118) along with the fluid outputs (116) surround the cutting or treatment tip.

11. The treatment device (110) as set forth in any of claims 1-10, wherein the cutting or treatment tip comprises one or more of a flashlamp and a laser diode.

12. The treatment device (110) as set in any of claims .1-11, wherein the number of illumination waveguides (118) is at least twice the number of fluid outputs (116).

13. A treatment device (110) as set forth in any of claims 1-12, wherein:
the cutting or treatment tip is configured to facilitate generation and emission in a distal direction, relative to the treatment device (110), of at least one output pulse, the output pulse travelling into a volume; and
the at least three fluid outputs (116) are configured to propagate fluid particles in the distal direction for reception by the propagating fluid particles of energy from the output pulse in the volume and expansion of the propagating fluid particles as a consequence of the reception.

14. The treatment device (110) as set forth in any of claims 1-11 and 13, wherein:
the sleeve is a cylindrically-shaped sleeve (138);
the cutting or treatment tiρ comprises a flashlamp;
each of the fluid outputs (116) is oriented to direct fluid particles into a path of the output pulse from the flashlamp to at least partially interact with the output pulse; and
disruptive forces are imparted to the target during use, the disruptive forces comprising one or more of disruptive cutting forces and disruptive ablating forces.

15. The treatment device (110) as set forth in any of claims 1-10 and 13, wherein:
the cutting or treatment tip comprises a laser diode;
each of the fluid outputs (116) is oriented to propagate fluid particles into a path of the output pulse from the laser diode to at least partially interact with the output pulse; and
disruptive forces are imparted, during use, to the target, the disruptive forces comprising one or more of disruptive cutting forces and disruptive ablating forces.

16. The treatment device (110) as set forth in any of claims 1-15, wherein the number of illumination waveguides (118) is greater than a number of the at least three fluid outputs (116).

17. The treatment device (110) as set forth in any of claims 1-16, wherein the fluid outputs (116) encircle the cutting or treatment tip and the plurality of waveguides (118) are circumferentially disposed relative to the cutting or treatment tip and concentrically disposed relative to the fluid outputs (116).

## Patentansprüche

1. Behandlungsvorrichtung (110), umfassend eine Anregungsquelle elektromagnetischer Energie zur Erleichterung der Durchführung eines Verfahrens an einem Ziel, umfassend:
a) eine Schneid- oder Behandlungsspitze,
b) einen oder mehrere Lichtübertragungseinrichtungen,
c) eine Hülse (138) mit optischen Wellenleitern (118), die die Schneid- oder Behandlungsspitze umgibt und die geeignet ist, um von der einen oder den mehreren Lichtübertragungseinrichtung(en) emittiertes Licht in eine Richtung zu leiten, die der Richtung von elektromagnetischer Energie entspricht, die durch die Schneid- oder Behandlungsspitze vom Ausgang distal von der Behandlungsvorrichtung (110) weg in ein in Bezug auf die Behandlungsvorrichtung (110) distal angeordnetes Volumen geleitet wird, um die Zieloberfläche zu beleuchten,
d) mindestens drei innerhalb der Hülse (138) angeordnete Fluidauslässe (116), die der Behandlungsvorrichtung (110) abgewandt und dem Volumen zugewandt sind und so ausgestaltet sind, dass sie Fluid distal von der Behandlungsvorrichtung (110) weg in Richtung des Volumens verbreiten, um dadurch das sich verbreitende Fluid in die elektromagnetische Energie innerhalb des Volumens einzubringen, während die elektromagnetische Energie distal von der Behandlungsvorrichtung weg in das Volumen geleitet wird.

2. Behandlungsvorrichtung (110) nach Anspruch 1, **dadurch gekennzeichnet, dass**:
die Fluidauslässe (116) um die Schneid- oder Behandlungsspitze herum in einer planaren Oberfläche, die senkrecht zu der Richtung der elektromagnetischen Energie verläuft, angeordnet sind und
die Schneid- oder Behandlungsspitze die elektromagnetische Energie relativ zu der Einbringung des sich verbreitenden Fluids abgibt, so dass sich die elektromagnetische Energie mit dem sich verbreitenden Fluid schneidet, und zwar in Form mindestens eines Ausgangsimpulses, der sich während der Verwendung mit dem sich verbreitenden Fluid in dem Volumen schneidet.

3. Behandlungsvorrichtung (110) nach Anspruch 1 oder 2, **dadurch gekennzeichnet, dass**:
die Fluidauslässe (116) um die Schneid- oder Behandlungsspitze herum auf einer senkrecht zu der Richtung verlaufenden Oberfläche angeordnet sind,
jeder der Fluidauslässe (116) so ausgerichtet ist, dass eine Längsachse parallel zu der Richtung der Abgabe von Fluidpartikeln entlang der Achse verläuft, und
die elektromagnetische Energie eine relativ große Energiemenge in zumindest einen Teil des sich verbreitenden Fluids in dem Volumen abgibt, wobei die relativ große Energiemenge, die in das sich verbreitende Fluid abgegeben wird, ausreicht, um das sich verbreitende Fluid dazu zu bringen, dass es expandiert und disruptive Schneid- oder Ablationskräfte auf das Ziel wirken lässt.

4. Behandlungsvorrichtung nach einem der Ansprüche 1 bis 3, **dadurch gekennzeichnet, dass** jeder der Mehrzahl von Beleuchtungswellenleitern (118) in der Hülse (138) benachbart zu einem der oder mehreren der Fluidauslässe (116) angeordnet ist.

5. Behandlungsvorrichtung (110) nach einem der Ansprüche 1 bis 4, **dadurch gekennzeichnet, dass**:
die Fluidauslässe (116) auf einer planaren Oberfläche (138) angeordnet sind und
die Oberfläche (138) die Mehrzahl von Wellenleitern (118) umfasst, wobei sich jeder Wellenleiter zwischen entsprechenden zwei Fluidauslässen (116) befindet.

6. Behandlungsvorrichtung (110) nach einem der Ansprüche 1 bis 5, **dadurch gekennzeichnet, dass**:
die Fluidauslässe (116) so ausgestaltet sind, dass sie Wasser in das Volumen einbringen, und
die Schneid- oder Behandlungsspitze elektromagnetische Energie aus einem Er:YAG-Laser, einem Er:YSGG-Laser, einem Er,Cr:YSGG-Laser oder einem CTE:YAG-Laser empfängt.

7. Behandlungsvorrichtung (110) nach einem der Ansprüche 1 bis 6, **dadurch gekennzeichnet, dass** die Mehrzahl von in der Hülse (138) angeordneten Wellenleitern (118) so ausgestaltet ist, dass sie das sichtbare Licht ausgibt, und die Fluidauslässe (116) so ausgestaltet sind, dass sie gleichzeitig mit dem Leiten von elektromagnetischer Energie aus der Schneid- oder Behandlungsspitze und dem Abstrahlen von Licht aus den Wellenleitern (118) Fluid in das Volumen einbringen.

8. Behandlungsvorrichtung (110) nach einem der Ansprüche 1 bis 7, **dadurch gekennzeichnet, dass** die elektromagnetische Energie aus der Schneid- oder Behandlungsspitze (a) eine Wellenlänge innerhalb eines Bereiches von ca. 2,69 bis ca. 2,80 Mikrometern oder (b) eine Wellenlänge von ca. 2,94 Mikrometern aufweist.

9. Behandlungsvorrichtung (110) nach einem der Ansprüche 1 bis 8, **dadurch gekennzeichnet, dass** die Fluidauslässe (116) peripher angeordnet sind und die Mehrzahl von Wellenleitern (118) peripher auf der Oberfläche zur Ausgabe von Licht angeordnet ist.

10. Behandlungsvorrichtung (110) nach einem der Ansprüche 1 bis 9, **dadurch gekennzeichnet, dass** die Mehrzahl von Wellenleitern (118) zusammen mit den Fluidauslässen (116) die Schneid- oder Behandlungsspitze umgibt.

11. Behandlungsvorrichtung (110) nach einem der Ansprüche 1 bis 10, **dadurch gekennzeichnet, dass** die Schneid- oder Behandlungsspitze eine Blitzlampe und/oder eine Laserdiode umfasst.

12. Behandlungsvorrichtung (110) nach einem der Ansprüche 1 bis 11, **dadurch gekennzeichnet, dass** die Anzahl der Beleuchtungswellenleiter (118) mindestens doppelt so groß wie die Anzahl der Fluidauslässe (116) ist.

13. Behandlungsvorrichtung (110) nach einem der Ansprüche 1 bis 12, **dadurch gekennzeichnet, dass**:
die Schneid- oder Behandlungsspitze so ausgestaltet ist, dass sie die Erzeugung und Emission mindestens eines Ausgangsimpulses in einer relativ zur Behandlungsvorrichtung (110) distalen Richtung erleichtert, wobei sich der Ausgangsimpuls in ein Volumen hineinbewegt, und
die mindestens drei Fluidauslässe (116) so ausgestaltet sind, dass sie Fluidpartikel in der distalen Richtung verbreiten, damit die sich verbreitenden Fluidpartikel Energie aus dem Ausgangsimpuls in dem Volumen aufnehmen und als Folge dieser Energieaufnahme expandieren.

14. Behandlungsvorrichtung (110) nach einem der Ansprüche 1 bis 11 und 13, **dadurch gekennzeichnet, dass**:
die Hülse eine zylindrisch geformte Hülse (138) ist,
die Schneid- oder Behandlungsspitze eine Blitzlampe umfasst,
jeder der Fluidauslässe (116) so ausgerichtet ist, dass er Fluidpartikel in einen Weg des Ausgangsimpulses aus der Blitzlampe leitet, damit die Fluidpartikel zumindest teilweise mit dem Ausgangsimpuls in Wechselwirkung treten, und
während der Verwendung disruptive Kräfte auf das Ziel übertragen werden, wobei die disruptiven Kräfte disruptive Schneidkräfte und/oder disruptive Ablationskräfte umfassen.

15. Behandlungsvorrichtung (110) nach einem der Ansprüche 1 bis 10 und 13, **dadurch gekennzeichnet, dass**:
die Schneid- oder Behandlungsspitze eine Laserdiode umfasst,
jeder der Fluidauslässe (116) so ausgerichtet ist, dass er Fluidpartikel in einen Weg des Ausgangsimpulses aus der Laserdiode leitet, damit die Fluidpartikel zumindest teilweise mit dem Ausgangsimpuls in Wechselwirkung treten, und
während der Verwendung disruptive Kräfte auf das Ziel übertragen werden, wobei die disruptiven Kräfte disruptive Schneidkräfte und/oder disruptive Ablationskräfte umfassen.

16. Behandlungsvorrichtung (110) nach einem der Ansprüche 1 bis 15, **dadurch gekennzeichnet, dass** die Anzahl der Beleuchtungswellenleiter (118) größer als die Anzahl der mindestens drei Fluidauslässe (116) ist.

17. Behandlungsvorrichtung (110) nach einem der Ansprüche 1 bis 16, **dadurch gekennzeichnet, dass** die Fluidauslässe (116) die Schneid- oder Behandlungsspitze umgeben und die Mehrzahl von Wellenleitern (118) relativ zur Schneid- oder Behandlungsspitze peripher und relativ zu den Fluidauslässen (116) konzentrisch angeordnet ist.

## Revendications

1. Dispositif de traitement (110) comprenant une source d'excitation d'énergie électromagnétique pour faciliter la performance d'un processus effectué sur une cible, comprenant :
(a) une extrémité de découpe ou de traitement ;
(b) un ou plusieurs émetteurs de lumière ;
(c) un manchon (138) ayant des guides d'onde optique (118) qui entoure l'extrémité de découpe ou de traitement et qui est capable de transmettre une lumière émise depuis les un ou plusieurs émetteurs de lumière dans une direction correspondant à celle de l'énergie électromagnétique dirigée par l'extrémité de découpe ou de traitement à partir de la sortie distalement loin du dispositif de traitement (110) et dans un volume disposé distalement loin du dispositif de traitement (110) pour éclairer la surface cible ;
(d) au moins trois sorties de fluide (116) agencées dans le manchon (138), dirigées loin du dispositif de traitement (110) vers le volume, et configurées pour propager du fluide distalement loin du dispositif de traitement (110) en direction du volume pour ainsi mettre le fluide se propageant dans l'énergie électromagnétique, dans le volume, lorsque l'énergie électromagnétique est dirigée distalement loin du dispositif de traitement dans le volume.

2. Dispositif de traitement (110) selon la revendication 1, dans lequel :
les sorties de fluide (116) sont disposées autour de l'extrémité de découpe ou de traitement sur une surface plane perpendiculaire à la direction de l'énergie électromagnétique ; et
l'extrémité de découpe ou de traitement émet l'énergie électromagnétique par rapport au positionnement du fluide se propageant de sorte que l'énergie électromagnétique recoupe le fluide se propageant, sous la forme d'au moins une impulsion de sortie qui pendant une utilisation recoupe le fluide se propageant dans le volume.

3. Dispositif de traitement (110) selon l'une des revendications 1 ou 2, dans lequel :
les sorties de fluide (116) sont disposées autour de l'extrémité de découpe ou de traitement sur une surface perpendiculaire à la direction ;
chacune des sorties de fluide (116) est orientée en ayant un axe longitudinal parallèle à la direction de sortie des particules de fluide le long de l'axe ; et
l'énergie électromagnétique applique une quantité d'énergie relativement grande dans au moins une partie du fluide se propageant dans le volume, la quantité d'énergie relativement grande appliquée dans le fluide se propageant étant suffisante pour amener le fluide se propageant à se déployer et appliquer des forces de découpe ou d'ablation disruptives sur la cible.

4. Dispositif de traitement selon l'une quelconque des revendications 1 à 3, dans lequel les plusieurs guides d'onde de lumière (118) du manchon (138) sont chacun disposés adjacents à une ou plusieurs des sorties de fluide (116).

5. Dispositif de traitement (110) selon l'une quelconque des revendications 1 à 4, dans lequel :
les sorties de fluide (116) sont agencées sur une surface plane (138) ; et
la surface (138) comprend les plusieurs guides d'onde (118), chacun étant espacé mutuellement entre deux sorties de fluide correspondantes (116).

6. Dispositif de traitement (110) selon l'une quelconque des revendications 1 à 5, dans lequel :
les sorties de fluide (116) sont configurées pour mettre de l'eau dans le volume ; et
l'extrémité de découpe ou de traitement reçoit une énergie électromagnétique en provenance d'un laser parmi un Er:YAG, un Er:YSGG, un Er,Cr:YSGG et un CTE:YAG.

7. Dispositif de traitement (110) selon l'une quelconque des revendications 1 à 6, dans lequel les plusieurs guides d'onde (118) disposés dans le manchon (138) sont configurés pour émettre la lumière visible, et les sorties de fluide (116) sont configurées pour mettre, simultanément avec l'orientation de l'énergie électromagnétique à partir de l'extrémité de découpe ou de traitement et l'émission d'une lumière à partir des guides d'onde (118), du fluide dans le volume.

8. Dispositif de traitement (110) selon l'une quelconque des revendications 1 à 7, dans lequel l'énergie électromagnétique provenant de l'extrémité de découpe ou de traitement a une longueur d'onde parmi (a) une longueur d'onde située dans une plage allant d'environ 2,69 à environ 2,80 microns et (b) une longueur d'onde d'environ 2,94 microns.

9. Dispositif de traitement (110) selon l'une quelconque des revendications 1 à 8, dans lequel les sorties de fluide (116) sont agencées circonférentiellement et les plusieurs guides d'onde (118) sont disposés circonférentiellement sur la surface pour émettre une lumière.

10. Dispositif de traitement (110) selon l'une quelconque des revendications 1 à 9, dans lequel les plusieurs guides d'onde (118) ainsi que les sorties de fluide (116) entourent l'extrémité de découpe ou de traitement.

11. Dispositif de traitement (110) selon l'une quelconque des revendications 1 à 10, dans lequel l'extrémité de découpe ou de traitement comprend une ou plusieurs parmi une lampe éclair et une diode laser.

12. Dispositif de traitement (110) selon l'une quelconque des revendications 1 à 11, dans lequel le nombre de guides d'onde de lumière (118) est au moins deux fois le nombre de sorties de fluide (116).

13. Dispositif de traitement (110) selon l'une quelconque des revendications 1 à 12, dans lequel :
l'extrémité de découpe ou de traitement est configurée pour faciliter la création et l'émission dans une direction distale, par rapport au dispositif de traitement (110), d'au moins une impulsion de sortie, l'impulsion de sortie se déplaçant dans un volume ; et
les au moins trois sorties de fluide (116) sont configurées pour propager des particules de fluide dans la direction distale pour réception par les particules de fluide se propageant d'une énergie provenant de l'impulsion de sortie dans le volume et expansion des particules de fluide se propageant en conséquence de la réception.

14. Dispositif de traitement (110) selon l'une quelconque des revendications 1 à 11 est 13, dans lequel :
le manchon est un manchon formé de manière cylindrique (138) ;
l'extrémité de découpe ou de traitement comprend une lampe éclair ;
chacune des sorties de fluide (116) est orientée pour diriger des particules de fluide dans un trajet de l'impulsion de sortie provenant de la lampe éclair pour au moins partiellement interagir avec l'impulsion de sortie ; et
des forces disruptives sont appliquées à la cible pendant une utilisation, les forces disruptives comprenant une ou plusieurs des forces de découpe disruptives et des forces d'ablation disruptives.

15. Dispositif de traitement (110) selon l'une quelconque des revendications 1 à 10 et 13, dans lequel :
l'extrémité de découpe ou de traitement comprend une diode laser ;
chacune des sorties de fluide (116) est orientée pour propager des particules de fluide dans un trajet de l'impulsion de sortie provenant de la diode laser pour au moins partiellement interagir avec l'impulsion de sortie ; et
des forces disruptives sont appliquées, pendant une utilisation, à la cible, les forces disruptives comprenant une ou plusieurs parmi des forces de découpe disruptives et des forces d'ablation disruptives.

16. Dispositif de traitement (110) selon l'une quelconque des revendications 1 à 15, dans lequel le nombre de guides d'onde de lumière (118) est plus grand que le nombre des au moins trois sorties de fluide (116).

17. Dispositif de traitement (110) selon l'une quelconque des revendications 1 à 16, dans lequel les sorties de fluide (116) encerclent l'extrémité de découpe ou de traitement et les plusieurs guides d'onde (118) sont disposés circonférentiellement par rapport à l'extrémité de découpe ou de traitement et disposés concentriquement par rapport aux sorties de fluide (116).
